Europäisches Patentamt

European Patent Office

Office européen des brevets

(11) Veröffentlichungsnummer: **0 306 824 B1**

(12) **EUROPÄISCHE PATENTSCHRIFT**

(45) Veröffentlichungstag der Patentschrift: **24.06.92**

(21) Anmeldenummer: **88114246.7**

(22) Anmeldetag: **01.09.88**

(51) Int. Cl.⁵: **A61K 37/02**, A61K 37/24, //(A61K37/02,33:14,31:195, 31:17),(A61K37/24,33:14, 31:195,31:17)

(54) **Stabilisierte Humanprotein-Präparate.**

(30) Priorität: **05.09.87 DE 3729863**

(43) Veröffentlichungstag der Anmeldung:
**15.03.89 Patentblatt 89/11**

(45) Bekanntmachung des Hinweises auf die Patenterteilung:
**24.06.92 Patentblatt 92/26**

(84) Benannte Vertragsstaaten:
**AT BE CH DE ES FR GB GR IT LI LU NL SE**

(56) Entgegenhaltungen:
**EP-A- 0 178 665**
**WO-A-86/05096**

(73) Patentinhaber: **BOEHRINGER MANNHEIM GMBH**
**Sandhofer Strasse 116**
**W-6800 Mannheim 31(DE)**

(72) Erfinder: **Woog, Heinrich, Dr.rer.nat.**
**Lindenstrasse 6**
**W-6941 Laudenbach(DE)**
Erfinder: **Gruber, Werner**
**Am Heiligenberg 12**
**W-6943 Birkenau(DE)**
Erfinder: **Markl, Hans-Jörg**
**Auf der Kreh 6**
**W-6701 Ellerstadt(DE)**
Erfinder: **Demmer, Fritz, Dr. rer. nat.**
**Kastanienweg 21**
**W-6945 Hirschberg-Leutershausen(DE)**

EP 0 306 824 B1

**Beschreibung**

Gegenstand der vorliegenden Erfindung sind stabile nicht immunogene, physiologisch gut verträgliche, gelöste oder gefriergetrocknete galenische Zubereitungen von Humanproteinen sowie Verfahren zu deren Herstellung, insbesondere von Erythropoietin.

Humanproteine sind körpereigene, nur in geringen Mengen vorkommende Proteine wie z.B. tissue plasminogen activator (t-PA), Streptokinase, Urokinase, Interferon, verschiedene colony stimulating factors (CSF) oder Erythropoietin (EPO). Am Beispiel des EPO, das bevorzugt in der Formulierung eingesetzt wird, soll die Erfindung näher erläutert werden.

Erythropoietin (EPO) ist ein Glycoprotein, welches die Bildung von Hämoglobin bzw. Erythrocyten im Knochenmark stimuliert. Dies Lipoprotein wird hauptsächlich in der Niere gebildet, findet sich in sehr geringen Mengen im Serum und wird unter physiologischen Bedingungen teilweise im Urin ausgeschieden.

Fehlen des EPO bei Niereninsuffizienz, bedingt mit einer renalen Anämie. Durch Gabe von EPO in physiologischen Mengen, d.h. wenigen Mikrogrammen, in einer oder mehreren Dosierungen, kann die Bildung von Erythrocyten in solchen Fällen wieder angeregt werden. Da der Körper bereits auf geringe Dosisänderungen empfindlich reagiert, muß die Dosierung exakt reproduzierbar sein. Üblicherweise wird EPO als wässrige Lösung entweder i.m. oder i.v. injiziert oder als Spray über die Nasenschleimhaut appliziert.

Es ist jedoch bekannt, daß EPO und zwar sowohl das zunächst aus Humanurin gewonnene Produkt (Miyake et al, J. Biol.Chem.Vol.25, 5558-5564, 1977) als auch die inzwischen gentechnologisch erzeugten Produkte (WO 85-02610) in wäßriger Lösung nicht stabil sind und selbst bei einer Lagerung bei -80°C größere Aktivitätsverluste auftreten. Diese beiden Produkte unterscheiden sich etwas im Glycosilierungsmuster und in der Aktivität, ein direkter Vergleich mit dem im Serum enthaltenen EPO is bisher nicht bekannt geworden.

Diese Aktivitätsverluste sind einerseits auf eine Zerstörung des EPO durch katalytische Effekte der Oberfläche der zur Aufbewahrung dienenden Ampullen durch Schwermetallspuren, Luftsauerstoff etc. zurückzuführen, andererseits aber auch durch eine Anlagerung der EPO-Moleküle an die Gefäßwand, wobei auch eine teilweise Denaturierung eintreten kann. Da, wie oben gesagt, in jeder Dosiereinheit nur wenige Mikrogramme enthalten sind, können die Verluste durch Adsorption bereits nach kurzer Aufbewahrungszeit beträchtlich sein.

In der EP-A 178 576 ist daher beschrieben worden, durch Zusatz von polymeren Verbindungen, wie Human- oder Rinderserumalbumin, Lecitin, Dextran, Cellulose, Polyethylenglycol etc. diese Anlagerung an die Gefäßwand zu inhibieren, und damit eine Wiederfindung von EPO zu 75-98 % nach ca. 2 h Lagerung bei 20°C zu erzielen, gegenüber nur 16 % ohne solchen Zusatz. Gemessen wurde dabei allerdings nur die Wiederfindung einer radioaktiven Markierung ($^{14}$C), so daß diese Versuche nichts über die Stabilisierung des EPO gegen Zersetzung aussagen.

Nach unseren Befunden ist eine Langzeitstabilisierung mit solchen Mitteln jedoch nicht zu erzielen, d.h. die EPO-Wirksamkeit im Maus-Test nimmt stark ab, zudem können diese Mittel bei der Injektion immunogene Reaktionen hervorrufen.

Aus der EP-A 178 665 sind weiterhin "Stabilisatoren", insbesondere für gefriergetrocknete EPO-Präparate bekannte. Neben den polymeren Substanzen PEG 4000, Gelatine und Dextran 40, sind verschiedene Zucker und Zuckeralkohole, Aminosäuren, anorganische Salze und Thiolverbindungen genannt. Kombinationen dieser Stoffe mit Humanserumalbumin, Gelatine und Dextran sind ebenfalls genannt. Auch in dieser Literaturstelle wird nur die Wiederfindung der Radioaktivitäten nach 2 Monaten Lagerung der gefriergetrockneten Produkte bestimmt. Diese wird mit 87-99 % angegeben, gegenüber 60 % ohne Zusatz. Da das lyophilisierte Material direkt nach der Herstellung als Standard benutzt wurde ist nicht angegeben, wie hoch die Aktivitätsverluste bei der Herstellung der Präparate sind. Auch diese Präparate weisen einen hohen Wirksamkeitsverlust im Maus-Test auf.

Es stellte sich daher die Aufgabe, ein gutverträgliches EPO-Präparat zu finden, welches lagerstabil ist, d.h. die in-vivo Wirksamkeit bewahrt, nicht zu Adsorptionen an den Ampullen- oder Spritzenwänden führt und leicht in eine injizierbare Form gebracht werden kann.

Überraschenderweise wird diese Aufgabe durch eine in den Ansprüchen näher gekennzeichnete Kombination verschiedener Komponenten gelöst. Wie unsere Versuche gezeigt haben, besitzen die Einzelstoffe diese Eigenschaften nicht oder nur in geringerem Ausmaß.

Entscheidend für die Stabilisierung ist der Zusatz von Harnstoff und verschiedenen Aminosäuren. Harnstoff wird in 5-50 g/l, vorzugsweise 10-15 g/l eingesetzt. Als Aminosäuren seien beispielsweise Glycin, L-Alanin, L-Arginin, L-Isoleucin, L-Leucin, L-2-Phenylalanin, L-Glutaminsäure oder L-Threonin genannt. Mischungen der verschiedenen Aminosäuren scheinen einen besonders vorteilhaften Effekt zu haben. Die

2

jeweiligen Aminosäuren werden in Mengen von 0,5-50 g/l, vorzugsweise 1-20 g/l eingesetzt, wobei die Gesamtmenge der Aminosäure vorzugsweise 5-25 g/l betragen soll.

Notwendig ist ferner ein physiologisch verträglicher Puffer, welcher in der für Injektionslösungen erforderlichen geringen Konzentrationen (ca. 20-100 mMol/l) den für EPO erforderlichen pH-Bereich von 6,5-7,4, insbesondere 7,0-7,2 einstellt. Außer Phosphatpuffern sind auch Glycinat, Carbonat, Citrat u.a. brauchbar, wobei außer Natrium- auch Kalium- oder Ammoniumionen als Gegenion dienen können. Eine Pufferung wird zusätzlich durch die enthaltenen Aminosäuren bewirkt.

Die Haftung des EPO an den Ampullenwandungen und Spritzen wird wesentlich herabgesetzt durch Zusatz geringer Mengen eines Detergens. Da das Präparat vorwiegend injiziert werden soll, muß dieser Stoff physiologisch, insbesondere i.v. verträglich sein. Konzentrationen von 0,05-5 g/l, insbesondere 0.1-0,5 g haben sich bewährt. Nichtionogene Netzmittel, wie die verschiedenen Polymacrogol-Arten, insbesondere Polyethylensorbitanlaurat (Tween® 20 oder 80) oder Sorbitantrioleat (Span® 35 oder 80) oder Glycerinoleylsäurepolyglycolether (Labrafil [R]) haben sich zu diesem Zweck bewährt, andere verträgliche Stoffe können jedoch gleichermaßen eingesetzt werden.

Um den Einfluß von Schwermetallionen - die fast unvermeidlich bei der Verarbeitung, z.B. aus den verwendeten Metallgeräten, eingeschleppt werden, auf das EPO zu verringern, hat es sich ferner bewährt, der Lösung noch 0,01 - 5 g/l eines löslichen Calciumsalzes, vorzugsweise etwa 0,02-0,2 g/l Calciumchlorid zuzusetzen. Andere physiologisch verträgliche Komplexbildner, beispielsweise Citrat, EDTA, NTA, Panthotenat können ebenfalls eingesetzt werden.

Als Lösungsmittel wird reines Wasser für Injektionszwecke verwendet, dem zur Einstellung der Isotonie noch 0,5-10 g/l Natriumchlorid oder entsprechende Stoffe, wie Mannit, Sorbit etc. zugesetzt werden.

Zur Herstellung der erfindungsgemäßen Präparate werden alle Hilfsstoffe in der notwendigen Menge Wasser gelöst, die EPO-Präparation, die eine Aktivität von ca. 100.000 bis 200.000 Units/mg Protein aufweisen sollte, zugemischt, in entsprechende Ampullen sterilfiltriert, eingefroren und schonend bei tiefen Temperaturen lyophilisiert. Die erhaltenen Präparate sind unter Stickstoff über 2 Jahre bei 0°C und über 1 Jahr bei Raumtemperatur haltbar. Beim Rekonstituieren mit Wasser lösen sie sich in wenigen Sekunden ohne Trübung auf und können so entweder direkt intravenös oder intramuskulär injiziert oder nach Verdünnung mit isotonischen Lösungen (z.B. Kochsalzlösung) infundiert werden.

Dem Einfriervorgang kommt eine besondere Bedeutung zu. Die Hilfsstoffe werden in Art und Menge so gewählt, daß der eutektische Punkt der einzufrierenden Lösung zwischen -50 bis -30°C liegt. Mit Hilfe eines computergesteuerten Optimierungsprogramms wurden folgende optimale Bedingungen für die 3 Phasen der Lyophilisation ermittelt:

Einfrierzeit 12 - 14 Stunden bei -40°C

Haupttrocknung:     Soletemperatur +10°C, Druck $10^{-1}$ mbar, Zeit 48-60 Stunden

Nachtrocknung:     Soletemperatur +20°C, Druck $10^{-3}$ mbar, Zeit 4-6 Stunden.

Hierbei ist es wesentlich mit Hilfe einer $\Delta$p-Messungsvorrichtung sowie einer Leitfähigkeitsmessung zu erkennen, wann die Haupttrocknung abgeschlossen ist, damit das zu lyophilisierende Produkt nicht zu schnell erwärmt wird und ein Auftauen der eingefrorenen Lösung und der damit verbundene Aktivitätsverlust vermieden wird.

Die eingesetzten Hilfsstoffe sind so ausgewählt worden, daß ein gleichmäßig strukturierter zu lyophilisierender Eiskörper zustande kommt und daß während der Lyophilisation ein poröses Gerüst (Kuchen) erhalten wird, aus dem eine optimale Sublimation des Eises, vor allem auch gegen Ende der Haupttrocknung, möglich ist. Die Nachtrocknung erfolgt wie oben genannt bei nur +20°C über 4-6 Std. Diese schonende Behandlung ist wichtig, da es sonst zu einem Verlust der Aktivität beim zu lyophilisierenden Gut kommt.

In der Regel haben die so lyophilisierten Produkte einen Wassergehalt von etwa 2-5 % nach Karl Fischer. Dieser Restwassergehalt hängt von Art und Menge der Hilfsstoffe ab, die in der betreffenden Rezeptur eingesetzt werden.

Die wäßrigen Lösungen des stabilisierten EPO können auch direkt in Ampullen abgefüllt werden und ohne Lyophilisation als gebrauchsfertige Form in den Verkehr gebracht werden. Die Haltbarkeit ist dadurch jedoch gegenüber dem Lyophilisat verkürzt auf ca. 1 Jahr bei 0°C und einige Monate bei Raumtemperatur.

Nachfolgende Beispiele sollen das oben geschilderte näher erläutern:

**Beispiel 1**

Erythropoietin 2000 Units Injektionstrockensubstanz
(Ansatz für 35.000 Flaschen)

In einem sterilen, mit Rührwerk versehenen 100 1 V2A-Doppelmantelkessel werden die Hilfsstoffe gelöst:

| Harnstoff | 700,0 g |
|---|---|
| Natriumchlorid | 70,0 g |
| Tween® 20 | 7,0 g |
| Natriumdihydrogenphosphat x 1$H_2O$ | 38,4 g |
| Dinatriumhydrogenphosphat x 2$H_2O$ | 350,0 g |
| Calciumchlorid x 2$H_2O$ | 8,4 g |
| Glycin | 105,0 g |
| L-Leucin | 140,0 g |
| L-Isoleucin | 140,0 g |
| L-Threonin | 35,0 g |
| L-Glutaminsäure | 35,0 g |
| L-Phenylalanin | 70,0 g |
| Wasser f. Injektionszwecke ad | 70,0 l |

Zu 30 l dieser Hilfsstofflösung werden 214,3 ml einer Erythropoietin-Rohstoffcharge mit einem EPO-Titter von 140.000 Units/l ml gegeben und zum Endvolumen von 35 l aufgefüllt und durchgerührt. Mit der restlichen Hilfsstofflösung wird das Filtrationssystem gespült. Die Ansatzlösung wird über ein Membranfilter 0,2 μm Porenweite sterilfiltriert. Die sterilfiltrierte Lösung wird zu 1 ml Injektionsflaschen unter aseptischen Bedingungen abgefüllt und unter folgenden Kriterien in einer Lyophilisationsanlage gefriergetrocknet:
Einfrierzeit 12 - 14 Stunden bei -40°C
Haupttrocknung:     Soletemperatur +10°C, Druck $10^{-1}$ mbar Zeit 48-60 Stunden
Nachtrocknung:      Soletemperatur +20°C, Druck $10^{-3}$ mbar Zeit 4-6 Stunden
Man erhält so eine voluminöse, offenporige Injektionstrockensubstanz, die mindestens 2 Jahre im Kühlschrank und 1 Jahr bei Raumtemperatur haltbar ist und sich innerhalb von einigen Stunden in 2 ml Wasser für Injektionszwecke ohne Trübung und frei von Partikeln löst.

**Beispiel 2**

Erythropoietin-Lyophilisat 200 Units
(Ansatz für 35.000 Flaschen)

| Erythropoietin | 46,7 ml = 7 Mio Units |
|---|---|
| Natriumchlorid | 100,0 g |
| Tween® 20 | 10,0 g |
| Natriumdihydrogenphosphat x 1$H_2O$ | 155,0 g |
| Dinatriumhydrogenphosphat x 2$H_2O$ | 500,0 g |
| Calciumchlorid x 2$H_2O$ | 10,0 g |
| Harnstoff | 1000,0 g |
| L-Leucin | 150,0 g |
| L-Threonin | 120,0 g |
| L-Phenylalanin | 165,0 g |
| Wasser f. Injektionszwecke ad | 70,0 l |

Die Hilfsstoffe werden in 70 l Wasser für Injektionszwecke gelöst und danach in 2 Portionen auf 35 l aufgeteilt. Die ersten 35 l werden mit der erforderlichen Menge EP0-Wirkstoff versetzt. Die zweiten 35 l werden zum Spülen des Filtrationssystems verwendet. Die Ansatzlösung wird über ein Membranfilter 0,2 μm Porenweite sterilfiltriert. Die sterilfiltrierte Lösung wird zu 1 ml Injektionsflaschen unter aseptischen Bedingungen abgefüllt und unter den gleichen Kriterien lyophilisiert wie im Beispiel 1 angegeben. Man erhält so ein weißes, poröses, in 2 ml Waser gut lösliches Lyophilisat, das 2 Jahre im Kühlschrank oder 1 Jahr bei Raumtemperatur ohne großen Aktivitätsverlust gelagert werden kann.

**Beispiel 3**

Erythropoietin Lyophilisat 1000 Units
(Ansatz für 35.000 Flaschen)

| Erythropoietin | 233,33 ml = 35 Mio Units |
|---|---|
| Natriumchlorid | 100,0 g |
| Tween® 20 | 12,0 g |
| Natriumdihydrogenphosphat x $H_2O$ | 140,0 g |
| Dinatriumhydrogenphosphat x $2H_2O$ | 450,0 g |
| Calciumchlorid x $2H_2O$ | 10,0 g |
| Harnstoff | 700,0 g |
| Glycin | 1050,0 g |
| L-Leucin | 92,0 g |
| L-Glutaminsäure | 103,0 g |
| L-Phenylalanin | 115,5 g |
| Wasser f. Injektionszwecke ad | 70,0 l |

Die Hilfsstoffe werden in 70 l Wasser für Injektionszwecke gelöst und danach in 2 Portionen auf 35 l aufgeteilt. Die ersten 35 l werden mit der erforderlichen Menge EP0-Wirkstoff versetzt. Die zweiten 35 l werden zum Spülen des Filtrationssystems verwendet. Die Ansatzlösung wird über ein Membranfilter 0,2 µm Porenweite sterilfiltriert. Die sterilfiltrierte Lösung wird zu 1 ml Injektionsflaschen unter aseptischen Bedingungen abgefüllt und unter den gleichen Kriterien lyophilisiert wie im Beispiel 1 angegeben. Man erhält so ein weißes, poröses, in 2 ml Waser gut lösliches Lyophilisat, das 2 Jahre im Kühlschrank oder 1 Jahr bei Raumtemperatur ohne großen Aktivitätsverlust gelagert werden kann.

**Beispiel 4**

Erythropoietin Lyophilisat 500 Units
(Ansatz für 35.000 Flaschen)

| Erythropoietin | 116,67 ml = 17,5 Mio Units |
|---|---|
| Natriumchlorid | 70,0 g |
| Tween® 20 | 7,0 g |
| Natriumdihydrogenphosphat x $H_2O$ | 38,5 g |
| Dinatriumhydrogenphosphat x $2H_2O$ | 490,0 g |
| Calciumchlorid x $2H_2O$ | 5,6 g |
| Harnstoff | 840,0 g |
| L-Leucin | 92,4 g |
| L-Glutaminsäure | 105,0 g |
| L-Phenylalanin | 119,0 g |
| Wasser f. Injektionszwecke ad | 70,0 l |

Die Hilfsstoffe werden in 70 l Wasser für Injektionszwecke gelöst und danach in 2 Portionen auf 35 l aufgeteilt. Die ersten 35 l werden mit der erforderlichen Menge EPO-Wirkstoff versetzt. Die zweiten 35 l werden zum Spülen des Filtrationssystems verwendet. Die Ansatzlösung wird über ein Membranfilter 0,2 µm Porenweite sterilfiltriert. Die sterilfiltrierte Lösung wird zu 1 ml Injektionsflaschen unter aseptischen Bedingungen abgefüllt und unter den gleichen Kriterien lyophilisiert wie im Beispiel 1 angegeben. Man erhält so ein weißes, poröses, in 2 ml Waser gut lösliches Lyophilisat, das 2 Jahre im Kühlschrank oder 1 Jahr bei Raumtemperatur ohne großen Aktivitätsverlust gelagert werden kann.

**Beispiel 5**

Erythropoietin Lyophilisat 750 Units
(Ansatz für 35.000 Flaschen)

| Erythropoietin | 175,0 ml = 26,25 Mio Units |
|---|---|
| Natriumchlorid | 100,0 g |
| Tween® 20 | 12,0 g |
| Natriumdihydrogenphosphat x $H_2O$ | 140,0 g |
| Dinatriumhydrogenphosphat x $2H_2O$ | 450,0 g |
| Calciumchlorid x $2H_2O$ | 10,0 g |
| Glycin | 1250,0 g |
| L-Isoleucin | 98,0 g |
| L-Glutaminsäure | 130,0 g |
| L-Phenylalanin | 145,0 g |
| Wasser f. Injektionszwecke ad | 70,0 l |

Die Hilfsstoffe werden in 70 l Wasser für Injektionszwecke gelöst und danach in 2 Portionen auf 35 l aufgeteilt. Die ersten 35 l werden mit der erforderlichen Menge EPO-Wirkstoff versetzt. Die zweiten 35 l werden zum Spülen des Filtrationssystems verwendet. Die Ansatzlösung wird über ein Membranfilter 0,2 µm Porenweite sterilfiltriert. Die sterilfiltrierte Lösung wird zu 1 ml Injektionsflaschen unter aseptischen Bedingungen abgefüllt und unter den gleichen Kriterien lyophilisiert wie im Beispiel 1 angegeben. Man erhält so ein weißes, poröses, in 2 ml Waser gut lösliches Lyophilisat, das 2 Jahre im Kühlschrank oder 1 Jahr bei Raumtemperatur ohne großen Aktivitätsverlust gelagert werden kann.

**Beispiel 6**

Um die Wirksamkeit verschiedener Stabilisatoren zu testen, wurde eine Standardrezeptur mit 1000 U EPO/ml, welche als Hauptstabilisator Harnstoff enthielt, mit Polyvinylpyrrolidon/Eiweiß bzw. verschiedenen Aminosäuren versetzt und die Produkte lyophilisiert. Die Ergebnisse sind in Tabelle 1 zusammengefaßt.

Die Stabilität von EPO wurde nach 6 Wochen Lagerung des Lyophili-sats bei 35°C bzw. 0°C im Maus-Milz-Test gemäß der Vorschrift von G.Krystal in Exp. Hematol. 11, 649-660 (1983) wie folgt bestimmt:

$B_6C_3F_1$-Mäusen, weiblich, Gewicht ca. 20 g(Zentralinstitut für Ver-suchstierkunde, Hannover) wird an zwei aufeinanderfolgenden Tagen 60 mg/kg Phenylhydrazinhydrochlorid i.p. injiziert. Nach 3 weiteren Tagen wird die Milz entnommen, die Milzzellen werden in sterilem Komplettmedium (Dulbecco Modified Eagle's Medium + 584,0 mg/l L-Glutamin + 0,1 m Mol/l 2-Mercaptoäthanol + 20 % fötales Kälberserum) suspendiert und auf $4 \times 10^6$ kernhaltige Zellen/ml verdünnt. Die Suspension, der zuvor die Testsubstanz bzw. der EPO-Standard in jeweils geeigneten Konzentrationen, gelöst in BSA-Puffer, zugesetzt wurden, wird in Mikrotiterplatten verteilt (0,2 ml/Vertiefung). Nach Inkubation (22 Stunden, 37°C, Luft + 15 % $CO_2$) werden 20 l ³H-Methyl-Thymidin-Lösung mit Ci pro Vertiefung zugegeben und erneut 2 Stunden bei 37°C inkubiert. Danach wird der Inhalt mit Hilfe eines Zell-Harvesters überführt und mit destilliertem Wasser gewaschen. Der Einbau von ³H-Thymidin wird mit einem β-Scintillationszähler bestimmt und gegen die Standardpräparate ausgewertet.

Als Working Standard wurde der "P009-EPO-Standard" von Genetics Institute, Cambridge, Massachusetts, USA, der 112 U EPO/ml (und 503 ng Protein/ml) enthielt (abgeglichen gegen den EPO-Referenz-Standard der WHO "International Reference Preparation of Erythropoietin, Human, Urinary for Bioassay (2nd I.R.P., established 1971)" verwendet. Die Standardkonzentrationen lagen im Bereich von 10-100 mU/ml.

Die auf ihre EPO-Aktivität zu testenden Lyophilisat wurden zunächst je Ampulle in 2 ml Wasser für Injektionszwecke gelöst, die weiteren Verdünnungen erfolgten - ebenso wie bei dem Working Standard - mit BSA-Puffer (8,75 g NaCl 1,95 g $CaCl_2$ x $2H_2O$, 1,00 g BSA (bovine serum albumin der Fa. Calbiochem), Wasser für Injektionszwecke ad 1 l). ³H-Methyl-Thymidin (spezifische Aktivität: 2 Ci/mmol) wurde von New England Nuclear bezogen.

**Beispiel 7**

In gleicher Weise wie in Beispiel 6 wurde eine etwas abgewandelte Stammrezeptur mit Harnstoff und verschiedenen Aminosäuren bzw. Gemischen versetzt. Zum Vergleich wurden zwei Mannithaltige Rezepturen, welche EP 178665 entsprechen, mitgeprüft. Die Ergebnisse sind in Tabelle 2 zusammengefaßt.

Tabelle 1

| Zusammensetzung mg/Fläschchen | 1 819892 G | a 819893 H | b 819894 I | ab 819895 K |
|---|---|---|---|---|
| Erythropoietin | 1000 U | 1000 U | 1000 U | 1000 U |
| Harnstoff | 10,0 | 10,0 | 10,0 | 10,0 |
| Natriumchlorid | 9,0 | 4,0 | 9,0 | 1,0 |
| Tween® 20 | 0,1 | 0,1 | 0,1 | 0,1 |
| Na-dihydrogenphosphat x $H_2O$ | 0,55 | 0,55 | 0,55 | 0,55 |
| Di-Na-hydrogenphosphat x2$H_2O$ | 5,0 | 5,0 | 5,0 | 5,0 |
| $CaCl_2$ x 2$H_2O$ | 0,08 | 0,08 | 0,08 | 0,08 |
| Kollidon 12 PF | 5,0 | - | 5,0 | - |
| Gelafundin | 1,0 | 1,0 | - | - |
| Glycin | - | 15,0 | - | 15,0 |
| L-Leucin | - | - | 2,0 | 2,0 |
| L-Isoleucin | - | - | 2,0 | 2,0 |
| L-Threonin | - | - | 0,5 | 0,5 |
| L-Glutaminsäure | - | - | 0,5 | 0,5 |
| L-Phenylalanin | - | - | 1,0 | 1,0 |
| Wasser | ad 1,0 ml | ad 1,0 ml | ad 1,0 ml | ad 1,0 ml |
| Stabilität 25°C | 493 | 869 | 934 | 1128 |
| Stabilität 0°C | 985 | 1114 | 1144 | 1205 |

## Tabelle 2

| Vers.-Nr. | 895 | 896 | 897 | 898 | 899 | 900 | 901 | 902 | 903 |
|---|---|---|---|---|---|---|---|---|---|
| **Zusammensetzung in mg/ml** | | | | | | | | | |
| EPO, Charge: P007 | 250 U | 250 U | 250 U | 250 U | 250 U | 250 U | 250 U | 250 U | 250 U |
| Natriumchlorid | 1,0 | 1,0 | 1,0 | 1,0 | 1,0 | 1,0 | 1,0 | 1,0 | 1,0 |
| Tween®20 | 0,1 | 0,1 | 0,1 | 0,1 | 0,1 | 0,1 | 0,1 | 0,1 | 0,1 |
| Na-Dihydrogenphosphat x $H_2O$ | 0,55 | 0,55 | 0,55 | 0,55 | 0,55 | 0,55 | 0,55 | 0,55 | 0,55 |
| DiNa-hydrogenphosphat x 2 $H_2O$ | 5,0 | 5,0 | 5,0 | 5,0 | 5,0 | 5,0 | 5,0 | 5,0 | 5,0 |
| Calciumchlorid x 2 $H_2O$ | 0,08 | 0,08 | 0,08 | 0,08 | 0,08 | 0,08 | 0,08 | 0,08 | 0,08 |
| Mannit | – | – | – | – | – | – | – | – | – |
| Harnstoff | – | 10 | – | 10 | – | 10 | – | 10 | – |
| Glycin | 15,0 | – | 15 | – | 15 | – | 15 | – | 15 |
| L-Leucin (1/100 m molar) | – | – | 1,32 | 1,32 | – | – | 1,32 | 1,32 | – |
| L-Isoleucin " | 1,32 | 1,32 | – | – | 1,32 | 1,32 | – | – | 1,32 |
| L-Threonin " | – | – | – | – | 1,2 | 1,2 | 1,2 | 1,2 | – |
| L-Glutaminsäure " | 1,47 | 1,47 | 1,47 | 1,47 | – | – | – | – | 1,47 |
| L-Phenylalanin " | – | – | – | – | – | – | – | – | 1,65 |
| L-Arginin " | 1,74 | 1,74 | 1,74 | 1,74 | 1,74 | 1,74 | 1,74 | 1,74 | – |
| $NaH_2PO_4$ x $H_2O$ | 1,0 | 1,0 | – | 1,0 | 2 | 2 | 2 | 2 | – |
| $Na_2HPO_4$ x 2 $H_2O$ | – | – | – | – | – | – | – | – | 2 |
| Wasser p.i. ad | 1,0 ml | 1,0ml | 1,0ml | 1,0ml | 1,0ml | 1,0ml | 1,0ml | 1,0ml | 1,0ml |
| Stabilität 25°C | 164 | 185 | 150 | 234 | 170 | 229 | 226 | 226 | 229 |
| Stabilität 0°C | 182 | 203 | 203 | 242 | 208 | 234 | 232 | 230 | 230 |

EP 0 306 824 B1

EP 0 306 824 B1

Tabelle 2   (Fortsetzung)

| Vers.-Nr. | 904 | 905 | 906 | 907 | 908 | 909 | 910 | 911 | 912 |
|---|---|---|---|---|---|---|---|---|---|
| Zusammensetzung in mg/ml | | | | | | | | | |
| EPO, Charge: P007 | 250 U | 250 U | 250 U | 250 U | 250 U | 250 U | 250 U | 250 U | 250 U |
| Natriumchlorid | 1,0 | 1,0 | 1,0 | 1,0 | 1,0 | 1,0 | 1,0 | 1,0 | 1,0 |
| Tween ®20 | 0,1 | 0,1 | 0,1 | 0,1 | 0,1 | 0,1 | 0,1 | 0,1 | 0,1 |
| Na-Dihydrogenphosphat x $H_2O$ | 0,55 | 0,55 | 0,55 | 0,55 | 0,55 | 0,55 | 0,55 | 0,55 | 0,55 |
| DiNa-hydrogenphosphat x 2 $H_2O$ | 5,0 | 5,0 | 5,0 | 5,0 | 5,0 | 5,0 | 5,0 | 5,0 | 5,0 |
| Calciumchlorid x 2 $H_2O$ | 0,08 | 0,08 | 0,08 | 0,08 | 0,08 | 0,08 | 0,08 | 0,08 | 0,08 |
| Mannit | - | - | - | - | - | - | - | - | - |
| Harnstoff | 10 | - | 10 | - | 10 | - | 10 | 10 | - |
| Glycin | - | 15 | - | 15 | - | 15 | - | 15 | 15 |
| L-Leucin(1/100 m molar) | - | 1,32 | 1,32 | - | - | 1,32 | 1,32 | 2 | - |
| L-Isoleucin          " | 1,32 | - | - | 1,32 | 1,32 | - | - | 2 | - |
| L-Threonin           " | - | - | - | 1,2 | 1,2 | 1,2 | 1,2 | 0,5 | - |
| L-Glutaminsäure      " | 1,47 | 1,47 | 1,47 | - | - | - | - | 0,5 | - |
| L-Phenylalanin       " | 1,65 | 1,65 | 1,65 | 1,65 | 1,65 | 1,65 | 1,65 | 1,0 | - |
| L-Arginin            " | - | - | - | - | - | - | - | - | - |
| $NaH_2PO_4$ x $H_2O$ | - | - | - | - | 1 | - | 1 | - | 1 |
| $Na_2HPO_4$ x 2 $H_2O$ | 5 | 7 | 5 | - | - | - | - | - | - |
| Wasser p.i. ad | 1,0 ml | 1,0ml | 1,0ml | 1,0ml | 1,0ml | 1,0ml | 1,0ml | 1,0ml | 1,0ml |
| Stabilität $25^{o}C$ | 205 | 221 | 258 | 205 | 216 | 171 | - | 256 | 175 |
| Stabilität $0^{o}C$ | 167 | 220 | 291 | 188 | 210 | 197 | 240 | 234 | 218 |

Tabelle 2 (Fortsetzung)

| Vers.-Nr. | 982 | 576 | 575 |
|---|---|---|---|
| Zusammensetzung in mg/ml | | | |
| EPO, Charge: P007 | 250 U | 250 U | 250 U |
| Natriumchlorid | 1,0 | 4 | 4 |
| Tween 20 | 0,1 | 0,1 | 0,5 |
| Na-Dihydrogenphosphat x $H_2O$ | 0,55 | 0,55 | 0,55 |
| DiNa-hydrogenphosphat x 2 $H_2O$ | 5,0 | 5,0 | 5,0 |
| Calciumchlorid x 2 $H_2O$ | 0,08 | 0,08 | 0,08 |
| Mannit | – | 20 | 30 |
| Harnstoff | 10 | – | – |
| Glycin | – | 10 | – |
| L-Leucin(1/100 m molar) | – | – | – |
| L-Isoleucin " | – | – | – |
| L-Threonin " | – | – | – |
| L-Glutaminsäure " | – | – | – |
| L-Phenylalanin " | – | – | – |
| L-Arginin " | – | – | – |
| $NaH_2PO_4$ x $H_2O$ | 1 | – | – |
| $Na_2HPO_4$ x 2 $H_2O$ | – | – | – |
| Wasser p.i. ad | 1,0ml | 1,0ml | 1,0ml |
| Stabilität 25°C | 120 | 134 | 97 |
| Stabilität 0°C | 195 | 172 | 178 |

## Patentansprüche

1. Verträgliches, lagerstabiles Humanprotein-Präparat, enthaltend ein Humanprotein, physiologisch verträgliche Puffer sowie gegebenenfalls Komplexbildner, Isotonie-einstellende Mittel, Calciumchlorid und andere für Injektionszwecke übliche Stoffe, dadurch gekennzeichnet, daß in der Injektionsform

5 - 50 g/l Harnstoff
0.5 - 50 g/l Aminosäure
0,05 - 5 g/l Detergens
enthalten ist.

2. Human-Präparat gemäß Anspruch 1, dadurch gekennzeichnet, daß das Detergens ein nichtionogenes Netzmittel ist.

3. Humanprotein-Präparat gemäß Anspruch 1, dadurch gekennzeichnet, daß die Aminosäure eine Mischung ist, die mindestens zwei der Aminosäure Glycin, L-Alanin, L-Isoleucin, L-Arginin, L-Leucin, L-2-Phenylalanin, L-Glutaminsäure oder L-Threonin enthält.

4. Humanprotein-Präparat nach Anspruch 2 oder 3, dadurch gekennzeichnet, daß als Netzmittel Polyethylensorbitanlaurat eingesetzt wird.

5. Humanprotein-Präparat nach einem der Ansprüche 1 - 4, dadurch gekennzeichnet, daß das Präparat in lyophilisierter Form vorliegt.

6. Humanprotein-Präparat nach einem der Ansprüche 1 - 5, dadurch gekennzeichnet, daß das Präparat in flüssiger, spritzfertiger Form vorliegt.

7. Humanprotein-Präparat nach einem der Ansprüche 1 - 6, dadurch gekennzeichnet, daß pro Injektionsdosiseinheit von 1 - 5 ml 100 - 1 Million U Humanprotein enthalten sind.

8. Humanprotein-Präparat nach einem der Ansprüche 1 - 7, dadurch gekennzeichnet, daß das Humanprotein Erythropoietin ist.

9. Humanprotein-Präparat nach Anspruch 8, dadurch gekennzeichnet, daß pro Injektionsdosiseinheit von 1 - 5 ml 100 - 20 000 U Erythropoietin enthalten sind.

10. Verfahren zur Herstellung von verträglichen, lagerstabilen Humanprotein-Präparaten, enthaltend ein Humanprotein, physiologisch verträgliche Puffer sowie gegebenenfalls Komplexbildner, Isotonie einstellende Mittel, Calciumchlorid und andere für Injektionszwecke übliche Stoffe, dadurch gekennzeichnet, daß man zunächst eine Lösung von 5 - 50 g/l Harnstoff, 0.5 - 50 g/l Aminosäure und 0,05 - 5 g/l Detergens herstelle, zu dieser Lösung übliche Hilfsstoffe fügt und anschließend das Humanprotein beimischt.

11. Verfahren gemäß Anspruch 10 dadurch gekennzeichnet, daß das Detergens ein nichtionogenes Netzmittel ist.

12. Verfahren zur Herstellung eines Präparates gemäß Anspruch 10, dadurch gekennzeichnet, daß das Humanprotein Erythropoietin ist.

13. Verfahren zur Herstellung eines Erythropoietin-Präparates gemäß Anspruch 10, 11 oder 12, dadurch gekennzeichnet, daß zunächst alle Hilfsstoffe in Wasser gelöst werden, das Erythropoietin zugegeben, die Mischung in Ampullen sterilfiltriert und schonend bei unter -20° C lyophilisiert wird.

14. Verfahren zur Herstellung eines Erythropoietin-Präparates gemäß Anspruch 10, 11 oder 12, dadurch gekennzeichnet, daß zunächst alle Hilfsstoffe in Wasser gelöst werden, das Erythropoietin zugegeben und die Mischung in Ampullen abgefüllt wird.

**Claims**

1. Compatible, storage-stable human protein preparation, containing a human protein, physiologically compatible buffer as well as possibly complex formers, isotonia-adjusting agents, calcium chloride and other materials usual for injection purposes, characterised in that, in the injection form, it contains 5 - 50 g/l urea, 0.5 - 50 g/l amino acid, 0.05 - 5 g/l detergent, conventional adjuvant materials are added to this solution and subsequently the human protein is admixed.

EP 0 306 824 B1

**2.** Human protein preparation according to claim 1, characterised in that the detergent is a non-ionogenic wetting agent.

**3.** Human protein preparation according to claim 1, characterised in that the amino acid is a mixture which contains at least two of the amino acids glycine, L-alanine, L-isoleucine, L-arginine, L-leucine, L-2-phenylalanine, L-glutamic acid or L-threonine.

**4.** Human protein preparation according to claim 2 or 3, characterised in that polyethylene sorbitan laurate is used as wetting agent.

**5.** Human protein preparation according to one of claims 1 - 4, characterised in that the preparation is present in lyophilised form.

**6.** Human protein preparation according to one of claims 1 - 5, characterised in that the preparation is present in liquid form ready for injection.

**7.** Human protein preparation according to one of claims 1 - 6, characterised in that, per injection dosage unit of 1 - 5 ml, it contains 100 to 1 million U of human protein.

**8.** Human protein preparation according to one of claims 1 - 7, characterised in that the human protein is erythropoietin.

**9.** Human protein preparation according to claim 8, characterised in that, per injection dosage unit of 1 - 5 ml, it contains 100 - 20,000 U or erythropoietin.

**10.** Process for the production of compatible storage-stable human protein preparations containing a human protein, physiologically compatible buffers as well as possibly complex formers, isotonia-adjusting agents, calcium chloride and other materials usual for injection purposes, characterised in that one first prepares a solution of 5 - 50 g/l urea, 0.5 - 50 g/l amino acid and 0.05 - 5 g/l detergent, adds usual adjuvants to this solution and subsequently admixes the human protein.

**11.** Process according to claim 10, characterised in that the detergent is a non-ionogenic wetting agent.

**12.** Process for the production of a preparation according to claim 10, characterised in that the human protein is erythropoietin.

**13.** Process for the production of an erythropoietin preparation according to claim 10, 11 or 12, characterised in that first all adjuvants are dissolved in water, the erythropoietin is added thereto, the mixture is sterile-filtered into ampoules and gently lyophilised at below -20°C.

**14.** Process for the production of an erythropoietin preparation according to claims 10, 11 or 12, characterised in that first all adjuvants are dissolved in water, the erythropoietin is added thereto and the mixture is filled into ampoules.

**Revendications**

**1.** Préparation de protéine humaine physiologiquement compatible, stable au stockage, contenant une protéine humaine, un tampon physiologiquement acceptable, ainsi qu'éventuellement un complexant, un agent d'ajustement isotonique, du chlorure de calcium et d'autres substances usuelles pour l'injection, caractérisée en ce que la forme galénique pour injection contient

    5 - 50 g/l d'urée
    0,5 - 50 g/l d'aminoacide
    0,05 - 5 g/l de détergent.

**2.** Préparation de protéine humaine selon la revendication 1, caractérisée en ce que le détergent est un agent mouillant non ionique.

**3.** Préparation de protéine humaine selon la revendication 1, caractérisée en ce que l'aminoacide est un

12

mélange contenant au moins deux aminoacides choisis parmi la glycine, la L-alanine, la L-isoleucine, la L-arginine, la L-leucine, la L-2-phénylalanine, l'acide L-glutamique ou la L-thréonine.

4. Préparation de protéine humaine selon la revendication 2 ou 3, caractérisée en ce que comme agent mouillant, on utilise le laurate de polyéthylènesorbitan.

5. Préparation de protéine humaine selon l'une quelconque des revendications 1 à 4, caractérisée en ce que la préparation se trouve sous forme lyophilisée.

6. Préparation de protéine humaine selon l'une quelconque des revendications 1 à 5, caractérisée en ce que la préparation se trouve sous forme liquide prête à l'injection.

7. Préparation de protéine humaine selon l'une quelconque des revendications 1 à 6, caractérisée en ce que chaque unité injectable de 1 - 5 ml contient 100 - 1 million d'unités de protéine humaine.

8. Préparation de protéine humaine selon l'une quelconque des revendications 1 à 7, caractérisée en ce que la protéine humaine est l'érythropoiétine.

9. Préparation de protéine humaine selon la revendication 8, caractérisée en ce que chaque unité injectable de 1 - 5 ml contient 100 - 20 000 unités d'érythropoiétine.

10. Procédé d'obtention de préparations de protéine humaine physiologiquement compatibles, stables au stockage, comprenant une protéine humaine, un tampon physiologiquement acceptable ainsi qu'éventuellement des complexants, un agent d'ajustement isotonique, du chlorure de calcium et d'autres substances usuelles pour l'injection, caractérisé en ce que l'on prépare d'abord une solution de 5 - 50 g/l d'urée, 0,5 - 50 g/l d'aminoacide et 0,05 - 5 g/l de détergent, à cette solution on ajoute les adjuvants usuels et ensuite, on la mélange avec la protéine humaine.

11. Procédé selon la revendication 10, caractérisé en ce que le détergent est un agent mouillant non ionique.

12. Procédé d'obtention d'une préparation selon la revendication 10, caractérisé en ce que la protéine humaine est l'érythropoiétine.

13. Procédé d'obtention d'une préparation d'érythropoiétine selon la revendication 10, 11 ou 12, caractérisé en ce que tous les adjuvants sont dissous dans l'eau, l'érythropoiétine est ajoutée, le mélange est conditionné en ampoules à travers un filtre stérile et lyophilisé avec précaution à une température inférieure à -20°C.

14. Procédé d'obtention d'une préparation d'érythropoiétine selon la revendication 10, 11 ou 12, caractérisé en ce que d'abord tous les adjuvants sont dissous dans l'eau, l'érythropoiétine est ajoutée et le mélange est conditionné en ampoules.